# EUROPEAN PATENT APPLICATION

(11) **EP 2 460 494 A1**
(43) Date of publication of application: **06.06.2012**
(21) Application number: 10193368.7
(22) Date of filing: 01.12.2010
(51) Int. Cl.: A61F 5/00, A61B 17/32, A61M 1/00, A61B 17/30, A61B 17/3205

(54) **Endoluminal sleeve cutting device and method for removing a lining from a hollow organ**

(71) Applicant: Ethicon Endo-Surgery, Inc., Cincinnati, Ohio 45242-2839 (US)
(72) Inventor: Cagle, David, Columbus, OH 43201 (US); Ortiz, Mark Steven, Milford, OH 45150 (US); D'Arcangelo, Michele, 00142 Roma (IT)
(74) Representative: Leihkauf, Steffen Falk

(57) **Abstract**

The present invention relates generally to medical apparatuses and methods and more particularly to devices and methods for cutting and removing a lining from a hollow body organ, such as a stomach, intestine, the gastrointestinal tract or any portion thereof.

In particular, the present invention relates to an endoluminal cutting device (1) for cutting a removing a portion of an endoluminal sleeve or lining (L) from a hollow body organ to which the said endoluminal sleeve or lining (L) is applied, the endoluminal cutting device (1) comprising a hollow flexible shaft (2) having a proximal end apt to be handled and operated by an external operator and a distal end (3) comprising a cutting assembly (4), wherein the said cutting assembly (4) comprises a tubular casing (5) that comprises lining acquisition means (8) and cutting means (7) in a reciprocally moveable relationship.

A method of using the said device is also described.

## Description

### FIELD OF THE INVENTION

The present invention relates generally to medical apparatuses and methods and more particularly to devices and methods for cutting and removing a lining from a hollow body organ, such as a stomach, intestine, the gastrointestinal tract or any portion thereof.

### BACKGROUND OF THE INVENTION

In cases of severe obesity, patients may currently undergo several types of surgery either to tie off or staple portions of the large or small intestine or stomach, and/or to bypass portions of the same to reduce the amount of food desired by the patient, and the amount absorbed by the gastrointestinal tract. The procedures currently available include laparoscopic banding, where a device is used to "tie off" or constrict a portion of the stomach, vertical banded gastroplasty (VBG), or a more invasive surgical procedure known as a Roux-En-Y gastric bypass to effect permanent surgical reduction of the stomach's volume and subsequent bypass of the intestine.

Although the outcome of these stomach reduction surgeries leads to patient weight loss because patients are physically forced to eat less due to the reduced size of their stomach, several limitations exist due to the invasiveness of the procedures, including time, general anesthesia, healing of the incisions and other complications attendant to major surgery. In addition, these procedures are only available to severely obese patients (morbid obesity, Body Mass Index >=40) due to their complications, including the risk of death, leaving patients who are considered obese or moderately obese with few, if any, interventional options.

In addition to the above described gastrointestinal reduction surgery, endoluminal sleeves are known for partially or totally lining certain portions of the stomach and of the intestine with the aim to separate or bypass at least part of the food flow from the lined portions of the gastrointestinal tract. It has been observed that by creating a physical barrier between the ingested food and certain regions of the gastrointestinal wall by means of endoluminal sleeves, similar benefits for weight loss and improvement or resolution of type 2 diabetes may be achieved as with gastric bypass surgery. Physicians believe that by creating a physical barrier between the ingested food and selected regions of the gastrointestinal wall, it might be possible to purposefully influence the mechanism of hormonal signal activation originating from the intestine.

A known type of endoluminal sleeve relies on metallic expandable structures, such as a stent, to engage the surrounding hollow organ for holding the sleeve in the planned position. To improve anchoring and stability of the sleeve, it is further known to provide the stent with barbs which penetrate the surrounding tissue.

This notwithstanding, it has been observed that the endoscopic sleeves tend to move inside the GI tract and migrate away from their initially planned position.

US patent n. 7,220,237 B2, Method and device for use in endoscopic organ procedures, to Gannoe et al. describes procedures for internally lining portions of the gastrointestinal tract, using tubular endoluminal sleeves and stapling devices for circumferentially acquiring tissue of the gastric wall and fixating a circular section of the acquired tissue to which an endoluminal sleeve is secured by shape interference.

However, the known methods and devices provide a permanent lining of the gastrointestinal tract. Even if, in some embodiments, the fastening means used for securing the endoluminal sleeve are reabsorbable, the healed tissue may tightly bind the lining, so that the endoluminal sleeve is retained over time.

This may cause a permanent incapacity of the patient to absorb the nutritious substances in the correct amount, leading to irreversible damages in extreme conditions.

Therefore, there is a need for improved devices and methods that allow the removal of an endoluminal sleeve or lining at a desired time.

Moreover, there is a need for devices and methods that avoid too invasive operations in order to prevent damages of the organ tissue which the lining is anchored to.

Moreover, there is a need for devices and methods that provide for the portionwise removal of the endoluminal sleeve, in order to tune the therapy according to the patient's needs and disease's evolution.

### SUMMARY OF THE INVENTION

The present invention provides for an improved apparatus and method for the transoral, or endoscopic, cutting and removal of an endoluminal lining within a hollow body organ, particularly the gastrointestinal tract, including, but not limited to, the esophagus, stomach, portions of or the entire length of the intestinal tract, etc., unless specified otherwise. In the case of the present invention, the surgeon or endoscopist may insert devices as described below through the patient's mouth, down the esophagus and into the stomach or intestine as appropriate. The procedure can be performed entirely from within the patient's stomach or other intestinal tract, and does not necessarily require any external incision.

At least part of the above identified needs are met by a method for removing a tubular sleeve or lining from a hollow body organ, particularly from a lumen of the gastrointestinal tract, to which the sleeve or lining has been anchored, comprising:
- providing an endoluminal cutting device comprising a hollow flexible shaft having a proximal end and a distal end, the distal end comprising a cutting assembly, wherein the cutting assembly comprises lining acquisition means and cutting means;
- introducing endoluminally the hollow flexible shaft of the endoluminal cutting device through the mouth of a patient, until the distal end thereof is positioned at a portion of the endoluminal sleeve or lining;
- operating the lining acquisition means to acquire a substantially circular portion of the lining;
- circularly cutting the lining at a point upstream the said acquired circular portion of the lining, with respect to the direction of introduction of the endoluminal cutting device;
- retracting the hollow flexible shaft of the endoluminal cutting device from the patient's mouth.

The method may encompass inspecting and identifying by an endoscope the area of the endoluminal sleeve or lining to be cut.

According to an embodiment, the distal end of the hollow flexible shaft of the endoluminal cutting device can be positioned close to the anchoring point of the lining, or at any other point, in order to provide for a partial removal of the lining. The method of the invention may include retracting the cut portion of the endoluminal sleeve or lining into the hollow flexible shaft of the endoluminal cutting device, in order to allow a clean and safe extraction of the same from the patient's body.

At least part of the above identified needs are also met by an endoluminal cutting device specifically adapted for removal of an endoluminal sleeve or lining anchored to the wall of a hollow body organ, the device comprising a hollow flexible shaft having a proximal end apt to be handled and operated by an external operator and a distal end comprising a cutting assembly, wherein the said cutting assembly comprises a tubular casing that comprises lining acquisition means and cutting means in a reciprocally moveable relationship.

In an embodiment, the lining acquisition means comprise a body internally connected with suction means and clamping means for the lining.

In another embodiment, the blade edge of the cutting means coincide with the edge of the tubular casing outlet and the said lining acquisition means comprise a lining acquisition head of cylindrical shape, the said lining acquisition head being provided with grasping means for the lining. The grasping means comprise a plurality of barbs, hooks, teeth or the like, that are apt to grasp or perforate the lining.

These and other aspects and advantages of the present invention shall be made apparent from the accompanying drawings and the description thereof, which illustrate embodiments of the invention and, together with the general description of the invention given above, and the detailed description of the embodiments given below, serve to explain the principles of the present invention.

### DESCRIPTION OF THE DRAWINGS

- Figure 1 illustrates an endoluminal sleeve or lining that is applied to a tract of the intestine;
- Figure 2 illustrates three different examples of positioning of an endoluminal sleeve or lining in gastro-intestinal (GI) tracts of a patient;
- Figure 3 illustrates a sectional perspective view of the anchoring portion of an endoluminal sleeve or lining in a GI tract;

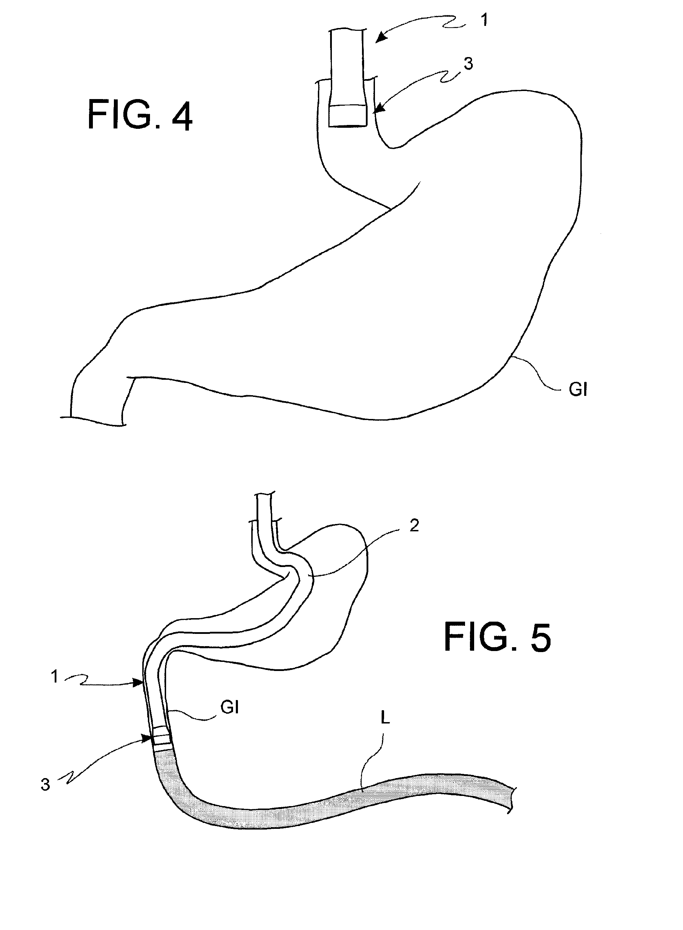

- Figure 4 illustrates a step of introduction of an endoluminal device in a GI tract, i.e. the cardia;
- Figure 5 illustrates a **further step** of introduction of an endoluminal device in a GI tract, i.e. the intestine, to reach the anchoring portion of an endoluminal sleeve or lining;
- Figure 6 shows a sectional view of a GI tract at the anchoring point of an endoluminal sleeve or lining, wherein a cutting device of the invention is shown during a step of clamping the lining;
- Figure 7 illustrates a sectional perspective view of the GI tract of figure 6, wherein the endoluminal sleeve or lining has been cut and removed;
- Figure 8 illustrates a perspective view of a different embodiment of the cutting device of the invention.

### DETAILED DESCRIPTION OF EMBODIMENTS

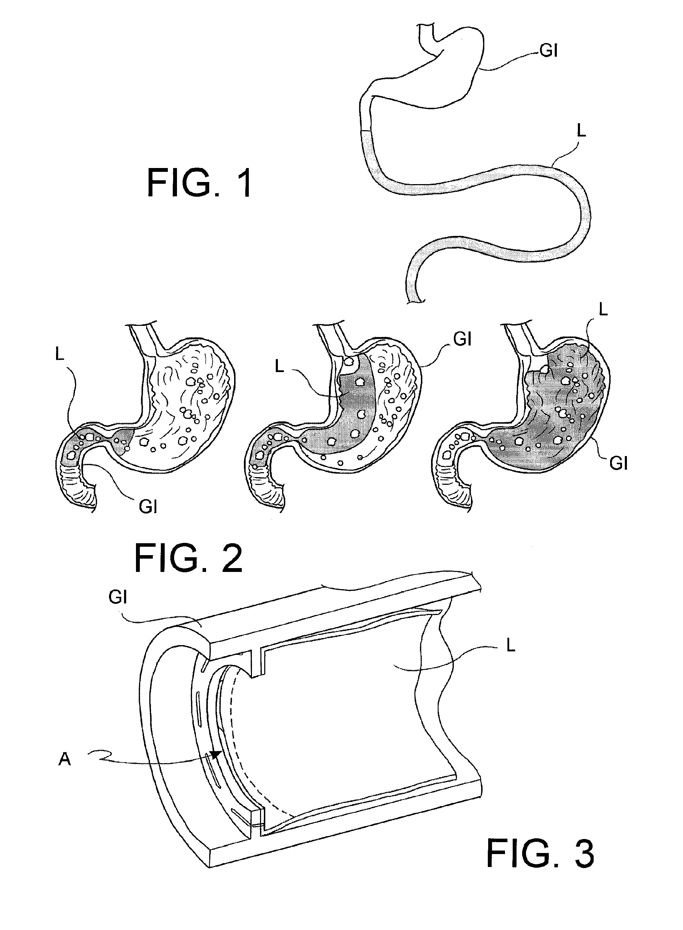

Referring to the drawings where like numerals denote like anatomical structures and components throughout the several views, figures 1 and 2 show an endoluminal sleeve or lining L applied to different portions of a hollow organ, i.e. a gastro-intestinal tract GI.

In figure 3, a particular of the anchoring portion A of the lining L is illustrated. The lining is anchored, for example, by stapling it to the tissue of the body organ.

The endoluminal cutting device of the invention, indicated with the numeral 1, comprises a hollow flexible shaft 2 having a proximal end (not shown) apt to be handled and operated by an external operator, i.e. a surgeon, and a distal end 3 comprising a cutting assembly 4.

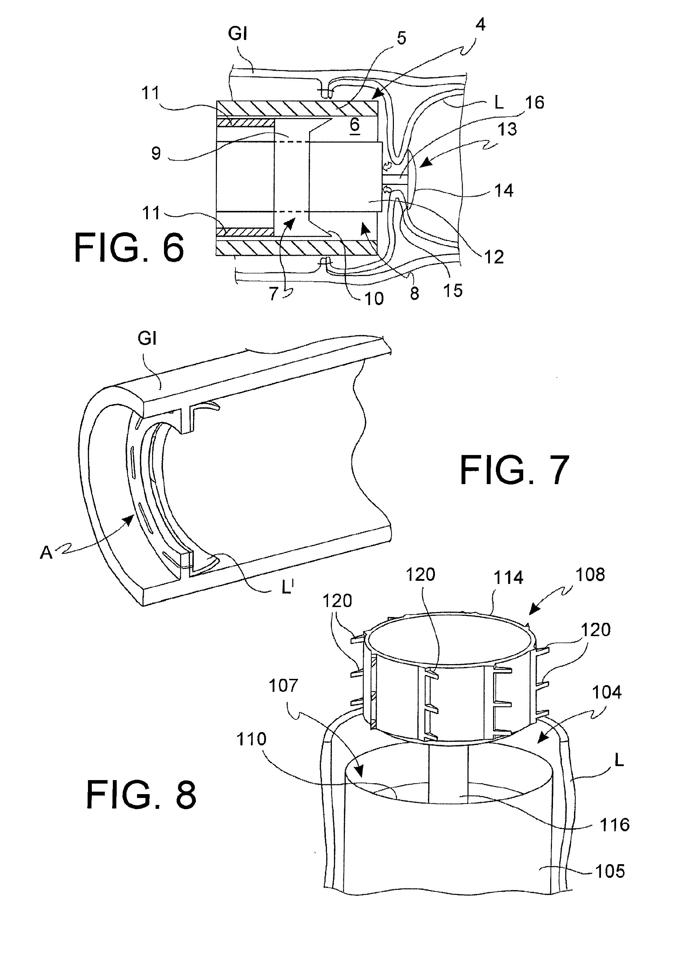

In the embodiment of figure 6, the cutting assembly 4 comprises a tubular casing 5, that may coincide with the distal end 3 of the hollow flexible shaft 2 or be a separate element. The tubular casing 5 will generally have a larger diameter with respect to the said hollow flexible shaft 2 and house a seat 6 wherein cutting means 7 and lining acquisition means 8 are accommodated in a moveable, i.e. slidable, relationship.

The cutting means 7 have a circular crown-shaped body 9 with a forward projecting blade edge 10. The blade edge 10 may have a continuous profile or an interrupted profile, i.e. a toothed profile. This latter embodiment will favor the penetration of the blade in the lining and the cutting action.

In an embodiment, the cutting means 7 have an external diameter that is less than or substantially matches the internal diameter of the tubular casing 5.

In an embodiment, the cutting means 7 are slidable between a retracted position (see figure 6) wherein they are housed inside the seat 6, and an advanced, operative position (not shown), wherein the cutting means 7 are brought to cut the lining L.

The cutting means 7 are operatively connected, through suitable actuating means 11, to an actuating mechanism (not shown) that allows the operation of the cutting means 7. In this embodiment, the lining acquisition means 8 are stationary or are slidable in a reciprocating direction, to match with the cutting means 7 at a point inside the casing 5.

In a different embodiment, the cutting means 7 are stationary, while the lining acquisition means 8 are slidable as will be described herein below.

The lining acquisition means 8 comprise a body 12 which is internally connected with suction means (not shown), for example surgical suction means of a conventional type.

The lining acquisition means 8 also comprise clamping means 13 for the lining. The clamping means 13 are mushroom-shaped and have a clamping head 14 of a generally circular shape, which comprises a sharp edge 15 apt to pinch the lining L. The clamping head 15 is connected to a stem 16 that is on its turn connected to actuating means (not shown) apt to move the clamping head 14 between a closure position - wherein the head 14 abuts against the body 12 and clamps the lining therebetween - and an open position - wherein the clamping head 14 is advanced to allow the lining to be sucked by the suctions means described above (see figure 6)-.

The lining acquisition means 8 are generally positioned in such a way that the body 12 ends at the outlet of the tubular casing 5, so that the clamping means 13 emerge from such an end. However, it is also possible that the body 12 protrudes from the outlet of the casing 5.

In the above embodiment wherein the lining acquisition means 8 are slidable, they can be operated between the said emerging position, i.e. a lining acquisition position - wherein they acquire the lining to be cut - and a retracted, cutting position - wherein the lining is pulled against the blade edge 10.

The diameter of the lining acquisition means 8, i.e. of the body 12 and the clamping head 14, is less than the diameter of the cutting means 7, so that they do not interfere each other during operation of the device.

In a different embodiment, illustrated in figure 8, a cutting assembly 104 is provided, that comprises a tubular casing 105 including cutting means 107 and lining acquisition means 108.

The cutting means 107 are circular crown-shaped, wherein the blade edge 110 corresponds to the edge of the tubular casing 105 outlet and can be obtained by beveling the terminal portion of the internal surface of the casing 105.

The lining acquisition means 108 comprise a lining acquisition head 114 of cylindrical shape, that is connected to a stem 116. The stem 116 is on its turn operatively connected to an actuating means (not shown) apt to move the head 114 between a lining acquisition position - wherein the lining acquisition head 114 is advanced to pick up the lining - and a retracted, cutting position - wherein the head 114 is pulled inside the tubular casing 105 and the acquired lining is cut while interfering with the blade edge 110 -.

The lining acquisition head 114 is provided with grasping means 120 for the lining. The grasping means 120 may comprise a plurality of barbs, hooks, teeth or the like, that are apt to grasp or perforate the lining, so that this latter is kept firmly when it is pulled toward the casing 105.

The said grasping means 120 can be fixed or retractable.

When the grasping means 120 are fixed, they can be made of rigid material or of a flexible material, such as nitinol. When the grasping means 120 are made of a rigid material, the overall diameter of the acquisition head 114 including the grasping means 120 must be the same as or less than the internal diameter of the tubular casing 105. Conversely, when the grasping means 120 are made of a flexible material, they may be configured in order to be bent against the surface of the head 114. In this configuration, the diameter of the acquisition head 114 can be increased to match substantially the internal diameter of the tubular casing 105, but for the residual thickness of the bent grasping means 120.

In another embodiment, as said above, the grasping means 120 are retractable. In this case, they may also be made of a rigid material. When the grasping means 120 are retractable, a suitable mechanism will be provided to move them between an extended condition (barbs, hooks or teeth protruding outwardly, as shown in figure 8) and a retracted condition, wherein they lie on the cylindrical surface of the acquisition head 114. For example, a cam or wedge mechanism of conventional type can be provided to push and pull the grasping means 120 outwardly and inwardly, respectively.

The cutting means 107, that have been described above to be integral with the edge of the tubular casing 105, may alternatively be provided internally to the casing, substantially in the same configuration described for the embodiment of figure 6.

Both in the embodiment of figure 6 and in the embodiment of figure 8, the endoluminal cutting device 1 is dimensioned to include a lumen for the introduction of an endoscope. In particular, the stem 16, 116 can be dimensioned in order to be hollow and to allow an endoscope to be inserted therein.

A further object of the present invention is a method for removing an endoluminal sleeve or lining from a hollow organ, specifically the gastro-intestinal tract or parts thereof, comprising the following steps:
a) providing an endoluminal cutting device 1 comprising a hollow flexible shaft 2 having a proximal end and a distal end 3, the distal end 3 comprising a cutting assembly 4, 104, wherein the cutting assembly 4, 104 comprises lining acquisition means 8, 108 and cutting means 7, 107;
b) introducing the hollow flexible shaft 2 of the endoluminal cutting device 1 through the mouth of a patient, until the distal end 3 thereof is positioned at a portion of the endoluminal sleeve or lining L;
c) operating the lining acquisition means 8, 108 to acquire a substantially circular portion of the lining L;
d) circularly cutting the lining L at a point upstream the said acquired circular portion of the lining, or in other words proximally, with respect to the direction of introduction of the endoluminal cutting device 1;
e) retracting the hollow flexible shaft 2 of the endoluminal cutting device 1 from the patient's mouth.

In an embodiment, step a) also encompasses inspecting and identifying by an endoscope the area of the endoluminal sleeve or lining L to be cut.

According to an embodiment, the distal end 3 of the hollow flexible shaft 2 is positioned just downstream the anchoring point A of the lining L, or in other words distally, with respect to the direction of introduction of the endoluminal cutting device 1. In this case, the cutting will occur very close to the anchoring point A, as depicted in figure 7. Nevertheless, it may be possible to cut the lining at any position downstream the anchoring point A, so that only part of the sleeve is removed. This may allow to tune the therapy according to the patient's need. According to an embodiment, the lining acquisition means 8 comprise clamping means 13. In this embodiment, step c) comprises sucking the lining L until a substantially circular section thereof is positioned at said clamping means 13 and subsequently clamping the sucked lining section with said clamping means 13; moreover, step d) comprises reciprocally moving the said lining acquisition means 8 with respect to said cutting means 7 until a circular section of the lining is cut.

The step of reciprocally moving the said lining acquisition means 8 with respect to said cutting means 7 as defined above may alternatively include: i) pulling backward the lining acquisition means 8 while the cutting means 7 are stationary, or ii) pushing forward the cutting means 7 while the lining acquisition means 8 are stationary, or iii) sliding both the cutting means 7 and the lining acquisition means 8 one toward each other.

According to another embodiment, the lining acquisition means 108 comprise grasping means 120. In this embodiment, step c) comprises moving forward the lining acquisition means 108 and optionally operating a revolutionary movement of the same to contact a substantially circular portion of the endoluminal sleeve L and to grasp the lining by the said grasping means 120; moreover, step d) comprises moving the lining acquisition means 108 backward until the blade edge 110 of the cutting means 107 circularly cuts the lining L.

In this latter embodiment, step c) can optionally include sucking the lining L through suitable suction means, such as a surgical suction tube, in order to detach the lining from the tissue of the hollow body organ which it is adhered to.

Moreover, in all embodiments depicted above, step d) can optionally include moving backward the lining acquisition means 8, 108 after the cut has been made, in order to retract the cut portion of the endoluminal sleeve or lining L into the hollow flexible shaft 2 of the device.

According to an alternative embodiment, the cut portion of the endoluminal sleeve or lining L is released from the lining acquisition means 8, 108 and is left to be eliminated by the patient's body through natural peristalsis. Releasing of the lining can be achieved by simply opening the clamping means 13 in the embodiment of figure 6 or scraping the surface of the cut lining portion against the internal surface of the tubular casing 105 in the embodiment of figure 8.

As clearly illustrated in the description above, the device and method of the present invention provide an easy and safe way to remove an endoluminal sleeve with minimally invasive impact to the patient.

The removal of the lining can be performed without the need of a surgical intervention, by a simple endoluminal operation.

It is also possible to remove only part of the endoluminal sleeve and to removing the remaining portion at a second time, to follow the progression of the therapy. The cut portion of the endoluminal sleeve can be retracted into the hollow flexible shaft of the instrument, so that it does not contact other parts of the patient's body which otherwise could be contaminated by its bacterial charge.

In some embodiments, the blade edge of the cutting means can be kept inside the tubular casing, so that the cutting operation is performed inside the casing thus avoiding the risk of cutting or damaging the tissue of the body organ.

Although preferred embodiments of the invention have been described in detail, it is not the intention of the applicant to limit the scope of the claims to such particular embodiments, but to cover all modifications and alternative constructions falling within the scope of the invention.

## Claims

1. An endoluminal cutting device (1) for cutting a removing a portion of an endoluminal sleeve or lining (L) from a hollow body organ to which the said endoluminal sleeve or lining (L) is applied, the endoluminal cutting device (1) comprising a hollow flexible shaft (2) having a proximal end apt to be handled and operated by an external operator and a distal end (3) comprising a cutting assembly (4, 104), wherein the said cutting assembly (4, 104) comprises a tubular casing (5, 105) having a distal opening, lining acquisition means (8, 108) and cutting means (7, 107), said lining acquisition means (8, 108) and cutting means (7, 107) being operable to move with respect to each other such that the cutting means cut the lining acquired by the lining acquisition means (8, 108).

2. The endoluminal cutting device (1) of claim 1, wherein the said lining acquisition means (8, 108) are moveable toward the cutting means (7, 107) and the said cutting means (7, 107) are stationary.

3. The endoluminal cutting device (1) of claim 1, wherein the said cutting means (7, 107) are moveable toward the lining acquisition means (8, 108) and the said lining acquisition means (8, 108) are stationary.

4. The endoluminal cutting device (1) of claim 1, wherein the said cutting means (7, 107) and the said lining acquisition means (8, 108) are moveable towards each other and are both moveable with respect to the casing.

5. The endoluminal cutting device (1) of any claim 1 to 4, wherein the said cutting means (7, 107) are circular crown-shaped and the said lining acquisition means (8, 108) have an external diameter which is less than the internal diameter of the cutting means (7, 107), so that the lining acquisition means (8, 108) can slide into the circular cutting means.

6. The endoluminal cutting device (1) of any claim 1 to 5, wherein the lining acquisition means (8) comprise a body (12) which is internally connected with suction means and clamping means (13) for clamping the lining.

7. The endoluminal cutting device (1) of claim 6, wherein the said clamping means (13) are mushroom-shaped and have a clamping head (14) of a generally circular shape, which comprises an edge (15) apt to pinch the endoluminal sleeve or lining (L) and wherein the clamping head (15) is connected to a stem (16) connected to actuating means apt to move the clamping head (14) between a closure position, wherein the clamping head (14) abuts against the body (12) and clamps the lining therebetween, and an open position, wherein the clamping head (14) is distally away from the body (12) to allow the lining to be sucked by the said suctions means.

8. The endoluminal cutting device (1) of claim 6 or 7, wherein the said lining acquisition means (8) are positioned in such a way that the body (12) ends at the distal opening of the tubular casing (5), so that the clamping means (13) emerge from said distal opening.

9. The endoluminal cutting device (1) of any claim 1 to 5, wherein a blade edge (110) of said cutting means (107) coincides with the edge of the distal opening of the tubular casing (105) and wherein the said lining acquisition means (108) comprise a lining acquisition head (114) of cylindrical shape, the said lining acquisition head (114) being provided with grasping means (120) for the lining.

10. The endoluminal cutting device (1) of claim 9, wherein the said grasping means (120) comprise a plurality of barbs apt to penetrate and grasp the lining.

11. The endoluminal cutting device (1) of claim 9 or 10, wherein the said grasping means (120) are stationary on the lining acquisition head (114) and the lining acquisition head (114) is retractable into the casing.

12. The endoluminal cutting device (1) of claim 11, wherein the said grasping means (120) are stationary and adapted to flex against an external surface of the lining acquisition head (114).

13. The endoluminal cutting device (1) of any claim 1 to 12, forming a lumen adapted to receive an endoscope.

14. A method for removing an endoluminal sleeve or lining (L) from a hollow organ, specifically the gastro-intestinal tract or parts thereof, comprising the following steps:
a) providing an endoluminal cutting device (1) comprising a hollow flexible shaft (2) having a proximal end and a distal end (3), the distal end (3) comprising a cutting assembly (4, 104), wherein the cutting assembly (4, 104) comprises lining acquisition means (8, 108) and cutting means (7, 107);
b) introducing endoluminally the hollow flexible shaft (2) of the endoluminal cutting device (1) through the mouth of a patient, until the distal end (3) thereof is positioned at a portion of the endoluminal sleeve or lining (L);
c) operating the lining acquisition means (8, 108) to acquire a substantially circular portion of the lining (L);
d) circularly cutting the lining (L) at a point upstream the said acquired circular portion of the lining (L), with respect to the direction of introduction of the endoluminal cutting device (1);
e) retracting the hollow flexible shaft (2) of the endoluminal cutting device (1) from the patient's mouth.

15. The method of claim 14, wherein step a) also encompasses inspecting and identifying by an endoscope the area of the endoluminal sleeve or lining (L) to be cut.

16. The method of claim 14 or 15, wherein the distal end (3) of the hollow flexible shaft (2) of the endoluminal cutting device (1) is positioned just downstream the anchoring point (A) of the lining (L), with respect to the direction of introduction of the endoluminal cutting device (1).

17. The method of any claim 14 to 16, wherein the lining acquisition means (8) comprise clamping means (13) and wherein step c) comprises sucking the lining (L) until a substantially circular section thereof is positioned at said clamping means (13) and subsequently clamping the sucked lining (L) section with said clamping means (13); an wherein step d) comprises reciprocally moving the said lining acquisition means (8) with respect to said cutting means (7) until a circular section of the lining (L) is cut.

18. The method of claim 17, wherein the step of reciprocally moving the said lining acquisition means (8) with respect to said cutting means (7) alternatively includes: i) pulling backward the lining acquisition means (8) while the cutting means (7) are stationary, or ii) pushing forward the cutting means (7) while the lining acquisition means (8) are stationary, or iii) sliding both the cutting means (7) and the lining acquisition means (8) one toward each other.

19. The method of any claim 14 to 16, wherein the lining acquisition means (108) comprise grasping means (120) and wherein step c) comprises moving forward the lining acquisition means (108) and optionally operating a revolutionary movement of the same to contact a substantially circular portion of the endoluminal sleeve or lining (L) and to grasp the lining by the said grasping means (120); and wherein step d) comprises moving the lining acquisition means (108) backward until the blade edge (110) of the cutting means (107) circularly cuts the lining (L).

20. The method of claim 19, wherein step c) includes sucking the lining (L) through suitable suction means, such as a surgical suction tube, in order to detach the lining from the tissue of the hollow body organ which it is adhered to.

21. The method of any claim 14 to 20, wherein step d) includes moving backward the lining acquisition means (8, 108) after the cut has been made, in order to retract the cut portion of the endoluminal sleeve or lining (L) into the hollow flexible shaft (2) of the endoluminal cutting device (1).

22. The method of any claim 14 to 20, wherein the cut portion of the endoluminal sleeve or lining (L) is released from the lining acquisition means (8, 108) and is left to be eliminated by the patient's body through natural peristalsis.
